# EUROPEAN PATENT APPLICATION

(11) **EP 0 628 533 A1**
(43) Date of publication of application: **14.12.1994**
(21) Application number: 93109098.9
(22) Date of filing: 07.06.1993
(51) Int. Cl.: C07C 51/09, C07C 67/03, C07C 231/02

(54) **Recovery of hydroxy acids from trash**

(71) Applicant: E.I. DU PONT DE NEMOURS AND COMPANY, Wilmington Delaware 19898 (US)
(72) Inventor: Brake, Loren Dale, South Wilmington, Delaware 19810 (US); Drysdale, Neville Everton, Newark, Delaware 19702 (US); Subramanian, Narayanan Samakara, Hockessin, Delaware 19707 (US)
(74) Representative: Jones, Alan John

(57) **Abstract**

This invention is a method for recovering hydroxy acid values from a poly(hydroxy acid) containing source, said method comprising, in order:
(a) contacting the source with a solubilizing fluid in an amount of at least 1 mole of solubilizing fluid per mole of hydroxy acid equivalent present in the source, said solubilizing fluid selected from the group consisting of (1) water, (2) alcohols containing 1 to 6 carbon atoms, (3) mixtures of water and said alcohols, (4) amines of the formula HNR₁R₂, wherein R₁ and R₂ are independently selected from the group consisting of hydrogen and alkyl groups of 1-4 carbon atoms; (5) liquid media containing diamines of the formula H(R₃)NR₅N(R₄)H, wherein R₃ and R₄ are independently selected from the group consisting of hydrogen and alkyl groups of 1-4 carbon atoms or are joined to form a heterocyclic ring, and R₅ is alkylene of 2-12 carbon atoms or phenylene; and (6) mixtures of said amines and/or diamines with water and/or alcohols containing 1 to 6 carbon atoms.

## Description

### Field of the Invention

This invention relates to the recovery of hydroxy acid value from a hydroxy acid polymer containing source, such as food container trash. Specifically, the invention relates to the recovery of high molecular weight poly(hydroxy acid) of at least 10,000 and normally 15,000 to 500,000 MW by at least partially depolymerizing and solubilizing the poly(hydroxy acid) in a solubilizing fluid.

### Background of the Invention

Shaped articles of high molecular weight (at least 10,000, and normally 15,000 to 500,000 MW) poly(hydroxy acids) (PHAs), particularly poly(lactic acid) (polylactide), poly(glycolic acid) (polyglycolide), and copolymers thereof, are well known. These polymers slowly hydrolyze and, thereafter, biodegrade to environmentally benign products. Consequently, high molecular weight PHA polymer shaped articles are replacing poly(styrene) and other non-degradable polymers to form products, such as fast food containers, that will degrade in a landfill (see, for example, Sinclair, W090/01521).

While this is a significant step in minimizing litter and long-term landfill disposal, discarding high molecular weight PHA articles has the cost penalty of discarding the valuable hydroxy acid (HA). Although the hydrolysis of PHAs is well known, heretofore it has not been achievable in a time frame to permit recovery and reuse of the hydroxy acid. Further, although high molecular weight PHAs are degradable, the time for degradation is so long as not to offer a significant lessening of the burden on landfills.

Thus, there is a need for an economical method to recover and recycle hydroxy acids from trash, both to recover the valuable hydroxy acid and to avoid burdening landfills with this waste.

### Summary of the Invention

The invention is a method for recovering hydroxy acid values from a poly(hydroxy acid) containing source, said method comprising, in order:
(a) contacting the source with a solubilizing fluid in an amount of at least 1 mole of solubilizing fluid per mole of hydroxy acid equivalent present in the source, said solubilizing fluid selected from the group consisting of (1) water, (2) alcohols containing 1 to 6 carbon atoms, (3) mixtures of water and said alcohols, (4) amines of the formula HNR₁R₂, wherein R₁ and R₂ are independently selected from the group consisting of hydrogen and alkyl groups of 1-4 carbon atoms; (5) liquid media containing diamines of the formula H(R₃)NR₅N(R₄)H, wherein R₃ and R₄ are independently selected from the group consisting of hydrogen and alkyl groups of 1-4 carbon atoms or are joined to form a heterocyclic ring, and R₅ is alkylene of 2-12 carbon atoms or phenylene; and (6) mixtures of said amines and/or diamines with water and/or alcohols containing 1 to 6 carbon atoms;
(b) maintaining the resulting mixture at sufficient temperature and pressure for a sufficient time to at least partially solubilize said polymer and form a liquid phase of enhanced monomer and/or oligomer hydroxy acid value; and
(c) isolating and recovering said liquid phase.

In one embodiment, the solubilizing fluid (1) is water, an alcohol containing 1 to 6 carbon atoms, or a mixture thereof, and (2) contains an effective amount of a strong acid. In another embodiment the poly(hydroxy acid) is selected from the group consisting of: poly(lactic acid); poly(glycolic acid); copolymers thereof; and polymers containing lactic acid and/or glycolic acid copolymerized with up to 30% of an additional monomer, said additional monomer selected from the group consisting of epsilon-caprolactone, delta-valerolactone, 1,5-dioxepen-2-one, 1,4-dioxan-2-one, beta-butyrolactone, and mixtures thereof.

### Detailed Description of the Invention

This invention is a method for economically and rapidly recovering the valuable PHA content of high molecular weight PHAs, thereby recycling instead of landfilling the PHA containing products. High molecular weight PHAs (molecular weight at least 10,000 and normally 15,000 to 500,000) are least partially depolymerized and solubilized by heating in a solubilizing fluid to form a liquid phase of enhanced monomer and/or oligomer hydroxy acid value. The solubilizing fluid is selected from the group consisting of: (1) water; (2) alcohols containing 1 to 6 carbon atoms; (3) mixtures of water and said alcohols; (4) amines of the formula HNR₁R₂, wherein R₁ and R₂ are independently selected from the group consisting of hydrogen and alkyl groups of 1-4 carbon atoms; (5) liquid media containing diamines of the formula H(R₃)NR₅N(R₄)H, wherein R₃ and R₄ are independently selected from the group consisting of hydrogen and alkyl groups of 1-4 carbon atoms and R₅ is alkylene of 2-12 carbon atoms or phenylene; and (6) mixtures of said amines and/or diamines with water and/or alcohols containing 1 to 6 carbon atoms.

This method can be used for the depolymerization of the high molecular weight PHAs. It is most useful for the depolymerization of poly(lactic acid), poly(glycolic acid), and copolymers of lactic and glycolic acid. It is also useful for PHAs of these monomers with other monomers. These copolymers preferably contain at least 70% of lactic acid and/or glycolic acid, and not more than 30% of the other monomer or monomers. Examples of other suitable monomers are: epsilon-caprolactone, delta-valerolactone, 1,5-dioxepan-2-one, 1,4-dioxan-2-one, beta-butyrolactone, beta-propiolactone, and 6-methyl-2,5-morpholinedione. Since the method has wide applicability in depolymerizing and recovering the monomer value of PHAs, the other monomers present in the PHA are not critical.

The PHA containing source can be any material that comprises PHA. It may consist essentially of PHA, such as PHA scrap, or the PHA may be mixed with other material, such as with paper and food residue in waste from a fast food restaurant.

The solubilizing fluid can be water, alcohols containing 1 to 6 carbon atoms, mixtures of water and said alcohols, amines of the formula HNR₁R₂, wherein R₁ and R₂ are independently selected from the group consisting of hydrogen and alkyl groups of 1-4 carbon atoms, and mixtures of said amines with water and/or alcohols containing 1 to 6 carbon atoms.

The solubilizing fluid can be water. Water includes any other medium that consists essentially of water, such as, for example, tap water. The PHA containing source is mixed with water and subjected to adequate heat and pressure to cause rapid and economical depolymerization. Preferably, the amount of water used will produce PHA depolymerization product (acid monomer and oligomers) at a concentration of at least 10%, and preferably greater than 70%, by weight. When adequate pressure is used, depolymerization conversion to the monomer and lower molecular weight oligomers in excess of 60%, and normally in the range of 70-100%, of theoretical is achieved.

The amount of water used affects both the time required to carry out the depolymerization and the percent conversion. At least one mole of water per mole of hydroxy acid equivalent present in the PHA source is required for depolymerization. Since an excess of water favors depolymerization, preferably a substantial excess is used, but not so much as to make product recovery an excessive expense. Normally a molar ratio of water to hydroxy acid equivalent present in the PHA is in the range of 1:1 to 5:1, preferably 1.5:1 to 2:1.

The solubilizing fluid may be an alkyl alcohol containing 1-6 carbon atoms. Preferred alkyl alcohols are: 2-propanol, 1-butanol, 2-methyl-2-butanol, 3-methyl-2-butanol, 3-pentanol, 2-pentanol, and 2-methyl-1-butanol. Because the resulting solution can be used directly for the preparation of PHA as described below, 1-butanol is more preferred.

The amount of alcohol used affects the time required to carry out the necessary solubilization and, if water is also present, depolymerization. At least one mole of alcohol per mole of hydroxy acid equivalent present in the PHA source is required for solubilization. Since an excess of alcohol favors solubilization, preferably a substantial excess is used, but not so much as to make product recovery an excessive expense. Normally a molar ratio of alcohol to hydroxy acid equivalent in the range of 1:1 to 5:1, preferably 1.5:1 to 2.5:1, is used.

In some cases, it may be desirable to add water to the alcohol to facilitate recovery and separation of the PHA reaction products from waste that is insoluble in alcohol and water. About 1 to 10 mole percent water, preferably 1 to 3 mole percent, relative to the amount of alcohol can be used. As is apparent to those skilled in the art, water can replace alcohol in the depolymerization reaction on a mole per mole basis. It should recognized that water may be present in the PHA containing source and may also cause depolymerization of the PHA on heating. If reaction of the PHA is undesirable, it may be necessary to dry the PHA containing source before treatment with the solubilizing fluid. When the alcohol/PHA source mixture is heated to the depolymerizing/solubilizing temperature in the presence of a small amount of water, a liquid phase that can readily be separated from the insoluble material by room temperature by filtration or the like is produced.

When water, alcohol, or mixtures thereof are used as the solubilizing fluid, various acids may be used to advantage as catalysts. In general strong organic or inorganic acids that do not react with the PHA to form undesirable by-products can be used. Liquid acids or water and/or alcohol soluble solid acids are preferred for ease of use and concentration. Sulfuric acid (6-36N) and and p-toluene sulfonic acid (solid) are excellent inexpensive acid catalysts. Methane sulfonic acid is also acceptable. Hydrochloric acid, although an effective catalyst, is generally undesirable because of its excessive corrosiveness to equipment.

Very small quantities of catalyst are required, normally in the weight range of 0.01-2%, preferably 0.1-0.5%, of the PHA. When an alcohol is used as the solubilizing fluid, depolymerization proceeds even if large quantities of water are introduced with the acid (i.e., a dilute, aqueous acid is added). However, this may be undesirable when the ester, rather than the acid, is the desired product.

When water is used as the solubilizing fluid, the solubilization product is low molecular weight oligomer, the average molecular weight depending on the solubilization conditions, i.e., reaction time, temperature, pressure, and the water/acid content of the solubilizing fluid.

The solubilizing fluid may be an ammonia or an alkyl amine. Useful amines are those of the formula HNR₁R₂ where R₁ and R₂ are independently selected from the group consisting of hydrogen and alkyl groups of 1-4 carbon atoms. Ammonia, methylamine, and dimethylamine are preferred.

The amine may be used neat or dissolved in water, an alkyl alcohols containing 1-6 carbon atoms, or a mixture thereof. In the preferred method using ammonia or an alkyl amine, aqueous ammonia is used as the solubilizing fluid for poly(lactic acid). An aqueous solution of lactamide [HOCH(CH₃)CONH₂] that can easily be separated from insoluble residues is formed.

The amount and type of amine used affects the time required to carry out the depolymerization and the percent conversion. Increased amine concentration increases the rate of depolymerization. Normally a molar ratio of amine to hydroxy acid equivalent present in the PHA containing source in the range of 1:1 to 2:1 is used. Since an excess of amine favors depolymerization, preferably a substantial excess is used, but not so much as to make amide recovery an excessive expense.

When depolymerization is completed to the desired extent, amide and any excess amine can be recovered by distillation, under vacuum, if necessary. When adequate amine is used, depolymerization conversion in excess of 60%, normally in the range of 70-90%, of theoretical is achieved, leaving a heel rich in PHA equivalents. If desired, this heel, with or without the addition of additional PHA containing source, can be sequentially reprocessed one or more times with further additions of amine. By this series of treatments, substantially 100% conversion of PHA to the amide can be achieved.

Diamines may be used in the solubilizing fluid. Useful diamines are those of the formula H(R₃)NR₅N(R₄)H, wherein R₃ and R₄ are independently selected from the group consisting of hydrogen and alkyl groups of 1-4 carbon atoms or are joined to form a heterocyclic ring, and R₅ is alkylene of 2-12 carbon atoms or phenylene. Typical alkylene groups are, for example, ethylene, propylene, hexamethylene, cyclohexylene, dodecylmethylene, etc. R₃ and R₄ may be joined to from a heterocyclic ring, for example, the piperazine ring.

In the preferred diamines R₃ and R₄ are each hydrogen. Preferred diamines are: 1,2-ethanediamine (ethylene diamine), 1,2-propanediamine, 1,3-propanediamine, 1,4-butanediamine, 1,5-pentanediamine, 1,6-hexanediamine, 1,7-heptaneamine, 1,8-octanediamine, 1,9-nonanediamine, 1,10-decanediamine, 1,11-undecanediamine, 1,12-dodecanediamine, cyclohexane-1,2-diamine, cyclohexane-1,3-diamine, cyclohexane-1,4-diamine, 1,3-bis(aminomethyl)cyclohexane, 1,4-bis(aminomethyl)cyclohexane, 1,3-bis(2-aminoethyl)cyclohexane, 1,4-bis(2-aminoethyl)cyclohexane, bis(4-aminocyclohexyl)methane, o-phenylene diamine, m-phenylene diamine, p-phenylene diamine, and piperizine. More preferred are 1,2-ethanediamine, 1,6-hexanediamine, 1,12-dodecanediamine, and bis(4-aminocyclohexyl)methane.

The diamine is present in a liquid medium. If the amine is a liquid, it may be used without addition of an added solvent. However, the reaction is typically run in a solvent. The solvent is preferably one that is non-reactive with and that does not dissolve the other materials present in the PHA containing source. In addition, the amine should be soluble in the solvent. Preferred solvents are acetonitrile and alcohols of 1-6 carbon atoms. If water is present in the PHA containing source, it may also act as a solvent.

The diaminediol formed from the reaction of the diamine with PHA can frequently be precipitated from the liquid phase formed by reaction of the solubilizing fluid with the PHA source by cooling the liquid phase from the reaction temperature to 0-40^{o}C. If the diaminediol is to be precipitated from the liquid phase, the insoluble material should be separated from the liquid phase by filtration or the like before the liquid phase is cooled.

Regardless of the nature of the solubilizing fluid, it is important to use temperatures and pressures adequate to cause rapid solubilization of the PHAs, but not severe enough to form undesirable degradation products. Higher temperature increases the rate of depolymerization and solubilization. By selecting optimal reaction conditions, particularly pressure and temperature, significant quantities of PHA can be adequately solubilized by a batch method, often in 1 hour and even in as little as 0.25 hour.

In many cases, overall economics and reaction kinetics dictate running the method at atmospheric pressure although elevated pressure sometimes is needed to reach the necessary temperatures for rapid and complete solubilization. Normally autogenous pressure is adequate. However, it may be desirable to use elevated pressures, up to about 70 kg/cm² (about 1000 psi) when high solubilization rates are desired.

A temperature in the range of 100-250^{o}C or higher, preferably 100-200^{o}C, is normally employed when water or an alcohol is the solubilizing fluid. A temperature in the range of 20-200^{o}C or higher, preferably 50-125^{o}C, is normally employed when ammonia or an alkyl amine is the solubilizing fluid. A temperature in the range of 40-150^{o}C is normally employed when a diamine is present in the solubilizing fluid.

When the 1-butanol (b.p. 118^{o}C) is the solubilizing fluid, increased pressure may not be necessary to attain rapid solubilization of the PHAs. However, with lower boiling lower alcohols, increased pressures may be required to attain rapid solubilization of the PHAs.

Reactor design, i.e., agitation, etc., also plays an important role in solubilization rate. Where speed is less a factor than other economies, batch times as long as 16 hour may be appropriate.

Continuous solubilization is also possible. In one such process PHA containing source and solubilization fluid flow through a multistage system in counter current fashion. The PHA containing source is continuously introduced into the first solubilization stage. Fresh solubilization fluid is continuously introduced into the last solubilization stage and the liquid phase formed in the solubilization reaction continuously recovered from the first stage.

When the desired amount of solubilization has occurred, insoluble material can be removed from the resulting liquid phase containing hydroxy acid, PHA oligomers, and/or other solubilization products, by a physical separation method, such as filtration, decantation, centrifugation, etc. The liquid phase can be recycled to PHA manufacture.

When water is the solubilizing fluid, the liquid phase can be used directly, or with some concentration, in the preparation of high molecular weight PHA using conventional processes. Discussions of processes for converting lactic acid, lactide (the cyclic dimer of lactic acid), and low molecular weight oligomers to polymer can be found in Bhatia, U.S. Patent 4,835,293, and Bellis, U.S. Patent 4,727,163. In the most economical routes to PHAs: (1) the hydroxy acid is converted to an ester; (2) the ester is dimerized to a cyclic dimer; and (3) the cyclic dimer is polymerized to PHA (See, for example, Bhatia, U.S. Patent 4,835,293; Bellis, U.S. Patent 4,727,163; Klootwijk, U.S. Patent 3,578,700; Hostettler, U.S. Patent 3,284,417; and De Vries, U.S. Patent 4,797,468.)

A discussion of processes for recovering lactic acid from aqueous solution, is found in Cockrem and Johnson, PCT Publication WO 93/00440 (PCT Application PCT/US92/04192). In a preferred method a solution of lactic acid in alcohol/water is acidified to a pH of about 1.0-1.5. The alcohol/water azetrope is removed by distillation. The pH is adjusted to pH 6.6-8.0 by the addition of an anhydrous base. The resulting mixture is filtered and washed with alcohol. The ester is recovered from the filtrate by distillation. 1-Butanol is the preferred alcohol for this method. This method is suited to recover hydroxy acids, particularly lactic acid, from water, alcohol, and/or water/alcohol solubilization reactions.

Polymeric materials may be prepared from the diaminediols formed when a diamine is used in the solubilizing fluid. This method is described in Barrows, U.S. Patents 4,343,931, and 4,529,792.

### Industrial Applicability

This invention provides an economical method to recover and recycle hydroxy acids from poly(hydroxy acid) containing material, such as trash, especially trash containing containers made from poly(hydroxy acids). This invention provides a method both to recover the valuable hydroxy acids and to avoid burdening landfills with this waste.

The advantageous properties of this invention can be observed by reference to the following examples which illustrate, but do not limit, the invention.

### EXAMPLES

Examples 1-14 illustrate the use of water as a solubilizing fluid.

### EXAMPLE 1

A mixture of 75 g polylactide (300,000 MW) and 38 g water was heated at 150^{o}C for 1 hr in a pressure vessel under autogenous internal pressure of about 7.7 kg/cm² (95 psig). The solid product depolymerized to lactic acid and low molecular weight oligomers to form an 80% fluid of the lactic acid values in water that can be easily pumped and transported for conversion to polymer for reuse.

### EXAMPLE 2

A mixture of 75 g polylactide and 50 g water was heated at 170^{o}C for 0.5 hr in a pressure vessel under autogenous internal pressure of about 8.8 kg/cm² (110 psig). The solid product depolymerized to lactic acid and low molecular weight oligomers in an aqueous fluid that can be pumped and transported for conversion to polymer for reuse.

### EXAMPLE 3-6

The method of Example 2 was repeated using the following ingredients with similar results.

| Example | Polymer |
|---|---|
| 3 | Copolymer of 80% lactic acid and 20% glycolic acid |
| 4 | Copolymer of 90% lactic acid and 10% glycolic acid |
| 5 | Copolymer of 80% lactic acid and 20% epsilon-caprolactone |
| 6 | Copolymer of 90% lactic acid and 10% beta-propiolactone |

### EXAMPLE 7

A mixture of 75 g polylactide (300,000 M.W.), 38 g water, and 0.5 g concentrated sulfuric acid was heated at 150^{o}C for 1 hr in a pressure vessel under an autogenous internal pressure of about 6.3 kg/cm² (75 psig). The product was a fluid than can be easily pumped and transported for conversion to polymer for reuse.

### EXAMPLE 8

A mixture of 75 g polylactide, 38 g water and 0.3 g p-toluene sulfonic acid was heated at 170^{o}C for 0.5 hr in a pressure vessel under autogenous pressure. The polylactide depolymerizes to lactic acid and low molecular weight oligomers as a liquid that can be pumped and transported for conversion to polymer for reuse.

### EXAMPLE 9

A mixture of 100 g polylactide, 50 g water and 0.2 g methane sulfonic acid was heated at 180^{o}C for 1 hr in a pressure vessel under autogenous pressure. The polylactide depolymerizes to lactic acid and low molecular weight oligomers aqueous liquid that can be pumped and transported for conversion to oligomer for reuse.

### EXAMPLES 10-14

These examples show the effect of reaction temperature on the solubilization reaction. A mixture of 75 g polylactide, 38 g water and 0.5 g concentrated sulfuric acid was in a pressure vessel under autogenous pressure under the conditions shown in the Table.

| Example | Conditions | Results |
|---|---|---|
| 10 | 100C/5 d | Trace solubilization |
| 11 | 130^{o}C/2 hr | Incomplete solubilization |
| 12 | 150^{o}C/2 hr | Complete solubilization |
| 13 | 50C/1 hr | Complete solubilization |
| 14 | 170^{o}C/0.5 hr | Complete solubilization |

Examples 15-37 illustrate the use of alcohols as a solubilizing fluid.

### EXAMPLE 15

A mixture of 100 g polylactide (300,000 MW), 200 g 1-butanol, 0.5 g brown bag, 2.0 g bread, 5.0 g sausage and 0.3 g wax paper was heated to 118^{o}C for two hr in a 0.5 L round bottom flask fitted with a reflux condenser. The resulting mixture was filtered thru a steam heated Buchner funnel to separate the insoluble material. The solids were washed with 50 g hot 1-butanol. The polylactide separated as a solid on cooling to room temperature and was isolated by filtration.

### EXAMPLE 16

A mixture of 75 g polylactide, 150 g 1-butanol and 0.5 g water was heated to 170^{o}C for 1 hr in a pressure vessel under autogenous pressure. The product was a liquid at room temperature and was easily pumped and transported for recovery and conversion to reusable polymer.

### EXAMPLE 17

A mixture of 75 g polylactide, 225 g 1-butanol and 2.0 g water was heated to 170^{o}C for 2 hr in a pressure vessel under autogenous pressure. The product was a liquid at room temperature and was easily pumped and transported for recovery and conversion to useable polymer.

### EXAMPLE 18

A mixture of 75 g polylactide and 150 g 1-butanol was heated to 115^{o}C for 0.5 hr. The hot mixture is a liquid. The polylactide separated as a solid on cooling to room temperature and was separated by filtration.

### EXAMPLE 19

A mixture of 100 g polylactide (300,000 MW), 200 g 1-butanol, 5 g water, 1.0 g brown paper bag, 3.0 g bread, 5.0 g sausage and 0.5 g wax paper was heated to 120^{o}C for 2 hr in a pressure vessel under autogenous pressure. The insoluble material were separated by filtration. The filtrate contained partially depolymerized polylactide and oligomers.

### EXAMPLE 20

Example 17 was repeated substituting 160 g 1-hexanol for the 1-butanol. Solid polylactide was separated on cooling to room temperature.

### EXAMPLES 21-25

The method of Example 18 was repeated using the following ingredients with similar results.

| Alcohol | Polymer |
|---|---|
| 1-Propanol | Copolymer of 80% lactic acid and 20% glycolic acid |
| Methanol | Copolymer of 90% lactic acid and 10% glycolic acid |
| 2-Propanol | Copolymer of 80% lactic acid and 20% epsilon caprolactone |
| 1-Butanol | Copolymer of 90% lactic acid and 10% beta-propiolactone |

### EXAMPLE 26

A mixture of 100 g polylactide (200,000 M.W.), 200 g 1-butanol, and 0.2 g p-toluene sulfonic acid was heated at 120^{o}C for 12 hr in a 0.5 L round bottom flask fitted with a reflux condenser. After the reaction mixture cooled to room temperature, 0.3 g sodium carbonate was added. The resulting mixture was distilled under vacuum to recover unreacted butanol (b.p. 66^{o}C at 80mmhg) and butyl lactate (b.p. 106^{o}C at 55 mmhg). Unconverted polylactide remains as the distillation heel. Recovered butyl lactate represented a 83% polylactide conversion.

### EXAMPLE 27

A mixture of 100 g polylactide (200,000 M.W.), 200 g 1-butanol, and 0.5 g concentrated sulfuric acid was heated at 120^{o}C for 12 hr. The reaction product was distilled under vacuum as described in Example 26 except that the sulfuric acid was not neutralized prior to distillation. Polylactide conversion to butyl lactate was 81%.

### EXAMPLE 28

A mixture of 100 g polylactide (200,000 M.W.), 200 g 1-butanol and 1.0 g concentrated sulfuric acid was heated at 120^{o}C for 5 hr in a 0.5 L round bottom flask fitted with a reflux condenser. The reaction product was distilled under vacuum as described in Example 27. The polylactide conversion to butyl lactate was 75%.

### EXAMPLE 29

This Example illustrates recycle of the distillation heel to the solubilization reaction step without addition of additional acid catalyst.

A mixture of 75 g polylactide (200,000 M.W.), 150 g 1-butanol and 0.1 g p-toluene sulfonic acid was heated at 120^{o}C for 3 hr in a 0.5 L round bottom flask fitted with a reflux condenser. The reaction product was distilled under vacuum as described in Example 27. The distillation heel weighed 60 g.

The distillation heel was mixed with 90 g 1-butanol and heated at 120^{o}C for 3 hr as described above. The reaction product was distilled under vacuum to recover the unconverted butanol and butyl lactate. The second distillation heel weighed 14 g.

The second distillation heel was mixed with 27 g 1-butanol and heated at 130^{o}C for 3 hr as described above. The reaction product was distilled under vacuum to recover the unconverted butanol and butyl lactate. The distillation heel weighed 3 g. Overall conversion of polylactide to butyl lactate was 97%.

Examples 30-33 illustrate the effect of reaction temperature on the solubilization reaction.

### EXAMPLE 30

A mixture of 75 g polylactide (200,000 M.W.), 150 g 1-butanol and 0.5 g concentrated sulfuric acid was heated at 120^{o}C for 2 hr in a 0.5 L round bottom flask fitted with reflux condenser at atmospheric pressure. The reaction product was cooled to room temperature and 0.7 g sodium carbonate was added to the reaction product. The resulting mixture was distilled under vacuum as described in Example 26. Conversion to butyl lactate: 36%.

### EXAMPLE 31

The procedure of Example 30 was repeated except that the solubilization reaction mixture was heated at 150^{o}C for 2 hr under autogenous pressure in a 0.4 L agitated pressure vessel. Conversion to butyl lactate: 69%.

### EXAMPLE 32

The procedure of Example 30 was repeated except that the solubilization reaction mixture was heated at 170^{o}C for 2 hr under autogenous pressure in a 0.4 L agitated pressure vessel. Conversion to butyl lactate: 84%.

### EXAMPLE 33

The procedure of Example 30 was repeated except that the solubilization reaction mixture was heated at 190^{o}C for 2 hr under autogenous pressure in a 0.4 L agitated pressure vessel. Conversion to butyl lactate: 83%.

### EXAMPLE 34

The procedure of Example 31 was repeated except that 96 g methanol was used in place of the 1-butanol. The reaction product was distilled at atmospheric pressure recover the unconverted methanol (b.p. 65^{o}C) and methyl lactate (b.p. 144^{o}C). Conversion to methyl lactate: 87%.

### EXAMPLE 35

The procedure of Example 31 was repeated except that 92 g ethanol was used in place of the 1-butanol. The reaction product was distilled at atmospheric pressure recover the unconverted ethanol (b.p. 79^{o}C) and ethyl lactate (b.p. 154^{o}C). Conversion to ethyl lactate: 78%.

### EXAMPLE 36

This Example illustrates recovery of polylactide from trash. A mixture of 100 g polylactide (300,000 M.W.), 200 g 1-butanol, 0.5 g brown bag, 2.0 g bread, 5.0 g sausage and 0.3 g wax paper was heated to 120^{o}C for 2 hr in a 0.5 L round bottom flask fitted with a reflux condenser. The resulting mixture was filtered hot through a steam heated Buchner funnel to separate the insoluble material. The solids are washed with 50 g hot 1-butanol. p-Toluene sulfonic acid (0.7 g) was added to the filtrate. The resulting solution was heated at 120^{o}C in a 0.5 L round bottom flask fitted with a reflux condenser for 11 hr. The reaction product was distilled under vacuum as described in Example 26. Conversion to butyl lactate: 55%.

### EXAMPLE 37

A mixture of 75 g polylactide (200,000 M.W), 150 g n-butanol, 20 g water and 0.5 g concentrated sulfuric acid was heated at 150^{o}C for 2 hr under autogenous pressure in a 0.4 L agitated pressure vessel. The reaction product was distilled at atmospheric pressure to remove the water and continued under vacuum as described in Example 26. Conversion to butyl lactate: 58%.

Examples 38-51 illustrate the use of liquids containing ammonia or an amine as the solubilizing fluid.

### EXAMPLE 38

A mixture of 75 g polylactide (300,000 MW) and 90 g of 60% dimethylamine in water was heated at 75^{o}C for 1 hr in a pressure vessel under autogenous pressure. The liquid product was distilled at atmospheric pressure to remove the water and continued under vacuum to give a 50% yield of N,N-dimethyl lactamide (b.p. 79.6^{o}C at 4mmhg).

### EXAMPLE 39

Example 38 was repeated using 60 g anhydrous dimethylamine in place of the 60% aqueous dimethylamine. Distillation gave a 75% yield to N,N-dimethyl lactamide.

### EXAMPLE 40

A mixture of 75 g polylactide, 50 g methanol and 50 g anhydrous methyl amine was heated at 75^{o}C for 0.75 hr in a pressure vessel under autogenous pressure. The liquid product was distilled to recover N-methyl lactamide.

### EXAMPLE 41

A mixture of 75 g polylactide (300,000 MW) and 100 g 28% ammonium hydroxide was heated for 1 hr in a pressure vessel under autogenous pressure at 75^{o}C. The product was a clear homogenous liquid consisting of lactic acid and soluble low molecular weight oligomers.

### EXAMPLES 42-45

The method of Example 39 was repeated using the following polymers. The results were similar to those of Example 39.
- 42.: Copolymer of 80% lactic acid and 20% glycolic acid
- 43: Copolymer of 90% lactic acid and 10% glycolic acid
- 44.: Copolymer of 80% lactic acid and 20% epsilon-caprolactone
- 45.: Copolymer of 90% lactic acid and 10% beta-propiolactone.

### EXAMPLES 46-49

The method of Example 40 was repeated using the indicated amines at the indicated reaction times and temperatures. The results were similar to those of Example 40.

| Example | Amine | Temperature | Hold Time |
|---|---|---|---|
| 46 | n-butyl amine | 150^{o}C | 0.5 hr |
| 47 | diethyl amine | 100^{o}C | 0.75 hr |
| 48 | i-propyl amine | 50^{o}C | 2 hr |
| 49 | n-propyl amine | 175^{o}C | 0.33 hr |

### EXAMPLE 50

A mixture of 30 g 1,2-ethanediamine, 50 mL acetonitrile, and 105 g of polylactide (MW 300,000) was heated at reflux for 2 hr in a stirred 0.5 L round bottom flask. After a hot filtration to remove insoluble material, the filtrate was cooled to 20^{o}C. The resulting N,N'-ethylenebislactamide was filtered. Yield: 83 g (80% based on diamine).

### EXAMPLE 51

A mixture of 58 g 1,6-hexanediamine, 50 mL acetonitrile, and 85 g of polylactide (MW 300,000) was heated at reflux for 3 hr in a stirred 0.5 L round bottom flask. After a hot filtration to remove insoluble material, the filtrate was cooled to 20^{o}C. The resulting N,N'-hexamethylenebislactamide was filtered.

Having described the invention, we now claim the following and their equivalents.

## Claims

1. A method for recovering hydroxy acid values from a poly(hydroxy acid) containing source, said method comprising, in order:
(a) contacting the source with a solubilizing fluid in an amount of at least 1 mole of solubilizing fluid per mole of hydroxy acid equivalent present in the source, said solubilizing fluid selected from the group consisting of (1) water, (2) alcohols containing 1 to 6 carbon atoms, (3) mixtures of water and said alcohols, (4) amines of the formula HNR₁R₂, wherein R₁ and R₂ are independently selected from the group consisting of hydrogen and alkyl groups of 1-4 carbon atoms; (5) liquid media containing diamines of the formula H(R₃)NR₅N(R₄)H, wherein R₃ and R₄ are independently selected from the group consisting of hydrogen and alkyl groups of 1-4 carbon atoms or are joined to form a heterocyclic ring, and R₅ is alkylene of 2-12 carbon atoms or phenylene; and (6) mixtures of said amines and/or diamines with water and/or alcohols containing 1 to 6 carbon atoms;
(b) maintaining the resulting mixture at sufficient temperature and pressure for a sufficient time to at least partially solubilize said polymer and form a liquid phase of enhanced monomer and/or oligomer hydroxy acid value; and
(c) isolating and recovering said liquid phase.

2. The method of Claim 1 wherein said poly(hydroxy acid) containing source is contaminated with or constitutes trash.

3. The method of Claim 2 wherein said liquid phase is isolated by filtration.

4. The method of Claim 1 wherein said solubilizing fluid is water, an alcohol containing 1 to 6 carbon atoms, or a mixture thereof, and said solubilizing fluid contains an effective amount of a strong acid.

5. The method of Claim 1 wherein said poly(hydroxy acid) is selected from the group consisting of: poly(lactic acid); poly(glycolic acid); copolymers thereof; and polymers containing lactic acid and/or glycolic acid copolymerized with up to 30% of an additional monomer, said additional monomer selected from the group consisting of epsilon-caprolactone, delta-valerolactone, 1,5-dioxepen-2-one, 1,4-dioxan-2-one, beta-butyrolactone, and mixtures thereof.

6. The method of Claim 1 or 5 wherein said temperature is 100-250^{o}C.

7. The method of Claim 1, 5, or 6 wherein said solubilizing fluid is water.

8. The method of Claim 7 wherein said solubilizing fluid contains an effective amount of a strong acid.

9. The method of Claim 7 or 8 additionally comprising, in order, following step (c):
(d) adding an alkyl alcohol of 4 or 5 carbon atoms to said liquid phase and adjusting the pH to about 1.0-1.5;
(e) removing the alcohol/water azetrope from said liquid phase by distillation;
(f) adjusting the pH of said liquid phase to pH 6.6-8.0; and
(g) recovering the lactate ester from said liquid phase by distillation.

10. The method of Claim 9 wherein said alcohol is 1-butanol.

11. The method of Claim 1, 5, or 6 wherein said solubilizing fluid is selected from the group consisting of alcohols containing 1 to 6 carbon atoms and mixtures of water and said alcohols.

12. The method of Claim 11 wherein said solubilizing fluid contains an effective amount of a strong acid.

13. The method of Claim 11 or 12 wherein said alkyl alcohol contains 4 or 5 carbon atoms and additionally comprising, in order:
(d) adjusting the pH of said liquid phase to about 1.0-1.5;
(e) removing the alcohol/water azetrope from said liquid phase by distillation;
(f) adjusting the pH of said liquid phase to pH 6.6-8.0; and
(g) recovering the lactate ester from said liquid phase by distillation.

14. The method of Claim 13 wherein said alcohol is 1-butanol.

15. The method of Claim 1 or 5 wherein said solubilizing fluid is selected from the group consisting of amines of the formula HNR₁R₂, wherein R₁ and R₂ are independently selected from the group consisting of hydrogen and alkyl groups of 1-4 carbon atoms, and mixtures of said amines with water and/or alcohols containing 1 to 6 carbon atoms.

16. The method of Claim 15 wherein said temperature is 20-200^{o}C.

17. The method of Claim 16 wherein R₁ and R₂ are each hydrogen.

18. The method of Claim 1 or 5 wherein said solubilizing fluid is selected from the group consisting of liquid media containing diamines of the formula H(R₃)NR₅N(R₄)H, wherein R₃ and R₄ are independently selected from the group consisting of hydrogen and alkyl groups of 1-4 carbon atoms or are joined to form a heterocyclic ring, and R₅ is alkylene of 2-12 carbon atoms or phenylene.

19. The method of Claim 18 wherein said temperature is 40-150^{o}C.

20. The method of Claim 19 wherein R₃ and R₄ are each hydrogen.

21. The method of Claim 20 additionally comprising, following step (c),
(d) cooling said liquid phase to precipitate the diamine/PHA reaction product.
